# EUROPÄISCHE PATENTSCHRIFT

(11) **EP 3 019 211 B1**
(45) Veröffentlichungstag und Bekanntmachung des Hinweises auf die Patenterteilung: **13.12.2017**
(21) Anmeldenummer: 14738388.9
(22) Anmeldetag: 07.07.2014
(51) Int. Cl.: A61M 1/10, A61M 1/16, A61M 1/36

(54) **BLUTBEHANDLUNGSVORRICHTUNG**
BLOOD TREATMENT DEVICE
DISPOSITIF DE TRAITEMENT SANGUIN

(30) Priorität: 09.07.2013 DE 102013011488; 26.07.2013 DE 102013012469
(43) Veröffentlichungstag der Anmeldung: 18.05.2016
(60) Teilanmeldung: 17192921.9
(73) Patentinhaber: Fresenius Medical Care Deutschland GmbH, 61352 Bad Homburg (DE)
(72) Erfinder: WIKTOR, Christoph, 63571 Gelnhausen (DE); HEIDE, Alexander, 65817 Eppstein (DE); PETERS, Arne, 61352 Bad Homburg (DE)
(74) Vertreter: Behr, Wolfgang
(86) Internationale Anmeldenummer: PCT/EP2014/001857
(87) Internationale Veröffentlichungsnummer: WO 2015/003794

(56) Entgegenhaltungen:
- WO-A1-02/064239
- WO-A1-2012/095294
- DE-A1-102009 060 668
- US-A- 4 293 961
- US-A- 4 968 422
- US-A- 5 417 648
- US-A1- 2005 101 901

## Beschreibung

Die Erfindung betrifft eine Blutbehandlungsvorrichtung.

Bei der Hämodialyse werden standardmäßig peristaltische Blutpumpen, die auch als Rollenpumpen bzw. Schlauchpumpen bezeichnet werden, zur Förderung des Bluts im extrakorporalen Blutkreislauf eingesetzt.

Die peristaltischen Blutpumpen okkludieren mit den Rollen ihres Rotors ein Pumpschlauchsegment des extrakorporalen Blutkreislaufs und erzeugen so eine pulsierende Strömung mit starken Druckpulsen und pulsierendem Fördervolumenstrom. So wird beispielsweise in der WO 97/10013 A1 ausführlich beschrieben, dass okkludierende Schlauchpumpen in der Praxis für die Hämodialyse üblich sind und dass die Druckpulse der okkludierenden Blutpumpe alle sonstigen Drucksignale im extrakorporalen Blutkreislauf im ungefilterten Drucksignal als Rauschen untergehen lassen.

Außerhalb des Gebiets der Hämodialyse sind für andere extrakorporale Blutkreisläufe auch Zentrifugal-Blutpumpen, die auch als Impeller-Blutpumpen bezeichnet werden, bekannt. Diese werden beispielsweise bei Herzoperationen eingesetzt.

Aus der DE102010007464A1 ist weiter bekannt, dass Impeller-Blutpumpen Bestandteile eines Disposables bzw. eines Blutschlauchsatzes, insbesondere auch einer Blutkassette sein können. Dort wird die Impellerpumpe dazu eingesetzt, um eine oder mehrere Membranpumpen anzutreiben.

Aus der WO2012/095294 A1 ist eine Blutbehandlungsvorrichtung gemäß dem Oberbegriff von Anspruch 1 bekannt. Dabei wird eine Blutbehandlungseinheit für eine extrakorporale Blutbehandlungsvorrichtung eingesetzt, deren Gehäuse sowohl eine Blutpumpe als auch eine semipermeable Membran aufnimmt. Bei der Blutpumpe handelt es sich um eine Impellerpumpe. Die Dialysevorrichtung weist eine Steuereinheit auf, die derart ausgebildet ist, dass die Blutpumpe in einem Pulsmodus betrieben werden kann, in welchem die Blutpumpe fortlaufend ein- und ausgeschaltet oder die Drehzahl der Pumpe fortlaufend verändert, d. h. erhöht und verringert wird. Der Betrieb der Blutpumpe im Pulsmodus soll die Diffusionsvorgänge an der Membran begünstigen.

Der Impeller einer Impeller-Blutpumpe ist in der Regel ohne unmittelbare mechanische Ankopplung - also berührungsfrei - magnetisch gelagert, so dass der Impeller lediglich mit dem geförderten Blut in reibungsbehafteten Kontakt tritt. Die Lagerung des Impellers kann auch eine Kombination aus magnetischer, hydraulischer und/oder mechanischer Lagerung sein.

Aufgabe der vorliegenden Erfindung ist es, eine verbesserte Blutbehandlungsvorrichtung konfiguriert zur Durchführung einer Hämodialyse und/oder Hämofiltration und/oder einer Hämodiafiltration mit einer Steuer- und Recheneinheit, die konfiguriert und/oder programmiert ist, eine Impeller-Blutpumpe anzusteuern, zur Verfügung zu stellen.

Erfindungsgemäß wird diese Aufgabe durch eine Blutbehandlungsvorrichtung gemäß Anspruch 1 der vorliegenden Erfindung gelöst.

Die vorliegende Erfindung beruht auf einem Verfahren zum Betrieb einer extrakorporalen Blutbehandlungsvorrichtung mit einem extrakorporalen Blutkreislauf für eine Hämodialyse und/oder Hämofiltration und/oder Hämodiafiltration, durch Ansteuerung einer Impeller-Blutpumpe, bei welchem die Impeller-Blutpumpe durch Hinzufügen eines pulsierenden Drehzahlanteils zu einer ersten konstanten Drehzahl pulsierend betrieben.

Hier macht sich das Verfahren den typischen Verlauf der Kennlinien von Impeller-Blutpumpen zunutze, welche das gezielte Vorgeben einer gewissen Druckpulsation durch pulsierende Erhöhung der Pumpendrehzahl über die normalen Betriebspunkte hinaus ermöglichen. Diese Eigenschaft wird bereits in der DE 10 2009 060 668 A1 und hier insbesondere in der Figur 2 gezeigt, in welcher die Druckerhöhung als Funktion des Blutflusses dargestellt ist.

Hierdurch ist es nun möglich, eine Pumpenpulsation vorzugeben, ohne dass der mittlere Durchfluss der Impeller-Blutpumpe nennenswert vom vorgegebenen Durchfluss bei konstanter Drehzahl abweicht. Eine gezielt vorgegebene Pumpenpulsation wiederum kann entsprechend der vorliegenden Erfindung einen verbesserten Betrieb des extrakorporalen Blutkreislaufs mit einer Impeller-Blutpumpe ermöglichen.

Insbesondere kann die Pumpenpulsation dabei ausgehend von gemessenen Druckpulswellen im extrakorporalen Blutkreislauf, wie etwa den durch Herzkontraktion verursachten Druckwellen des Patienten, vorgegeben werden.

Dabei kann erfindungsgemäß der Verlauf der Druckamplitude an mindestens einer Druckmessstelle des extrakorporalen Blutkreislaufs gemessen werden. Bevorzugt wird die Pumpenpulsation dabei ausgehend diesem gemessenen Verlauf der Druckamplitude im extrakorporalen Blutkreislauf vorgegeben.

Insbesondere kann dabei der Verlauf einer Druckamplitude an mindestens einer Druckmessstelle des extrakorporalen Blutkreislaufs gemessen werden, wobei der gemessene Verlauf der Druckamplitude in einen Soll-Wert für die Steuerung des pulsierenden Drehzahlanteils der Impellerpumpe eingeht. Dabei kann das Messsignal der Druckmessstelle direkt als Soll-Wert herangezogen werden, wofür es gegebenenfalls durch einen Tiefpass gefiltert und/oder skaliert wird. Der pulsierende Drehzahlanteil kann dabei anhand des Soll-Werts gesteuert und/oder über eine Rückkopplung geregelt werden.

Dabei kann der gemessene Verlauf ein Summensignal aus den durch den arteriellen und den venösen Patientenanschluss zum Drucksensor gelangenden Druckpulsen vom Herz des Patienten darstellen. Dies ist dadurch bedingt, dass keine okkludierenden Elemente und insbesondere keine okkludierenden Pumpen eingesetzt werden.

Erfindungsgemäß kann weiterhin die Impellerpumpe oder Zentrifugalpumpe zwischen dem Drucksensor und dem venösen Patientenzugang angeordnet sein, und/oder der Drucksensor in der venösen Tropfkammer angeordnet sein.

Erfindungsgemäß wird der pulsierende Betrieb der Impeller-Blutpumpe mit einem Verlauf der durch Herzkontraktion verursachten Druckpulswellen des Patienten im extrakorporalen Blutkreislauf synchronisiert. Insbesondere kann dies durch Heranziehen eines den Patientenpuls kennzeichnenden Parameters und/oder des oben bereits beschriebenen Messsignals erfolgen.

Zur Synchronisation kann ausgehend vom Betrieb der Impeller-Blutpumpe mit einer konstanten Pumpendrehzahl die Pumpendrehzahl zunächst um eine bestimmte erste Drehzahldifferenz abgesenkt und die so reduzierte Pumpendrehzahl wird mittels einer zweiten, jedoch mit bestimmter Frequenz pulsierend auf und abschwellenden Drehzahldifferenz erhöht. Damit kann das Blut im extrakorporalen Blutkreislauf mit pulsierendem Druckverlauf synchron zum Herzschlag des Patienten gefördert werden.

Der mittlere Durchfluss der Impellerpumpe im extrakorporalen Blutkreislauf bleibt dabei unverändert (z. B. 500 ml/min). Die Entnahme von Blut aus dem Patientenzugang (Fistel oder Shunt oder Graft) ist durch den pulsatilen, synchronisierten Betrieb der Impellerpumpe angepasst an das Angebot von Blut in dem Patientenzugang, d. h. bei großem Angebot wird viel entnommen und umgekehrt.

Gemäß einer weiteren Ausgestaltung der Erfindung wird diese Synchronisation derart ausgeführt, dass bei maximalem Druck das Blut im Patientenzugang, d. h. der Fistel angesaugt wird. Hierbei ist zu berücksichtigen, dass es bekannt ist, dass es im Patientenzugang, der am Patienten als Fistel oder Shunt angelegt sein kann, zu einer unerwünschten Fistelzirkulation von bereits behandeltem Blut kommen kann, wenn von der Blutpumpe über die arterielle Nadel mehr Blut in den extrakorporalen Blutkreislauf angesaugt wird als aufgrund des Blutflusses in der Fistel zur Verfügung steht. Der Fehlbetrag des Blutflusses wird nämlich durch Ansaugen des über die venöse Nadel in die Fistel zurückgeleiteten bereits dialysierten Bluts ausgeglichen. Dies wiederum führt zu einer Verminderung der Effizienz der Behandlung, da bereits dialysiertes Blut im extrakorporalen Blutkreislauf unmittelbar erneut dialysiert wird.

Erfindungsgemäß kann dabei der pulsierende Betrieb der Impeller-Blutpumpe mit dem Verlauf der durch Herzkontraktion verursachten Druckpulswellen des Patienten im extrakorporalen Blutkreislauf synchronisiert werden, so dass die Fistelrezirkulation des Blutes im extrakorporalen Blutkreislauf ein Minimum annimmt.

Das erfindungsgemäße Verfahren kann dabei folgende Schritte umfassen:
- Betreiben der extrakorporalen Blutbehandlungsvorrichtung mit dem extrakorporalen Blutkreislauf für eine Hämodialyse und/oder Hämofiltration und/oder Hämodiafiltration, durch Ansteuerung der Impeller-Blutpumpe zum Betrieb mit einer ersten konstanten Drehzahl,
- Bestimmen eines den Patientenpuls kennzeichnenden Parameters
- Pulsierendes Betreiben der Impeller-Blutpumpe durch Hinzufügen eines pulsierenden Drehzahlanteils zu einer ersten konstanten Drehzahl, wobei durch Heranziehen des den Patientenpuls kennzeichnenden Parameters der pulsierende Betrieb der Impeller-Blutpumpe mit dem Verlauf der durch Herzkontraktion verursachten Druckpulswellen des Patienten im extrakorporalen Blutkreislauf synchronisiert wird.

Das erfindungsgemäße Verfahren kann ebenfalls folgende Schritte umfassen, welche bevorzugt dem erfindungsgemäßen pulsierenden Betrieb vorangehen:
- Betreiben der extrakorporalen Blutbehandlungsvorrichtung mit dem extrakorporalen Blutkreislauf für eine Hämodialyse und/oder Hämofiltration und/oder Hämodiafiltration, durch Ansteuerung der Impeller-Blutpumpe zum Betrieb mit einer ersten konstanten Drehzahl,
- Messen mindestens eines ersten Verlaufs der Druckamplitude an mindestens einer Druckmessstelle des extrakorporalen Blutkreislaufs und
- Extrahieren des Verlaufs mindestens einer durch Herzkontraktion verursachten Druckpulswelle des Patienten im extrakorporalen Blutkreislauf aus dem ersten Verlauf der gemessenen Druckamplitude.

Der so extrahierte Verlauf mindestens einer durch Herzkontraktion verursachten Druckpulswelle des Patienten im extrakorporalen Blutkreislauf kann dabei zur Ansteuerung des erfindungsgemäßen pulsierenden Betriebes herangezogen werden bzw. als der den Patientenpuls kennzeichnende Parameter verwendet werden.

Die Extraktion des Verlaufs mindestens einer durch Herzkontraktion verursachten Druckpulswelle des Patienten im extrakorporalen Blutkreislauf aus dem ersten Verlauf der gemessenen Druckamplitude im Sinne der vorliegenden Erfindung muss dabei keinen zusätzlichen Schritt der Auswertung umfassen. Vielmehr kann als durch Herzkontraktion verursachte Druckpulswelle des Patienten im extrakorporalen Blutkreislauf der Verlauf der gemessenen Druckamplitude direkt - gegebenenfalls nach einer Tiefpass-Filterung und/oder Skalierung - herangezogen werden.

Bevorzugt wird die Steuerung der Impellerpumpe direkt mittels eines Regelkreises an das - gegen Rauschen eventuell Tiefpass-gefilterte - gemessene Signal des Drucksensors gekoppelt. Somit ist die Frequenz der Pumpenmodulation direkt durch das Messsignal vorgegeben.

Die Amplitude der Blutpumpenmodulation kann mit einem konstanten Faktor, z.B. 1, an die Amplitude des Drucksignals gekoppelt sein. Bevorzugt ist dieser Faktor in der Steuerung hinterlegt.

Dabei kann eine Auswertung des Signals im Hinblick auf die Erkennung bestimmter Zustände unterbleiben.

In alternativen Ausführungsformen können anstelle des Messens der Druckamplitude an mindestens einer Druckmessstelle des extrakorporalen Blutkreislaufs ergänzend oder ersatzweise auch andere den Patientenpuls kennzeichnende Parameter für die Synchronisation der Impellerpumpe oder Zentrifugalpumpe herangezogen werden. Alternativ kann es sich bei dem den Patientenpuls kennzeichnenden Parameter daher auch um Messwerte aus EKG, Pulsuhr, Blutdruckmanschette, Ultraschallmessung und/oder Flussmessung handeln.

Das erfindungsgemäße Verfahren kann ebenfalls folgende Schritte umfassen:
- Messen des Transmembrandrucks in der extrakorporalen Blutbehandlungsvorrichtung und
- pulsierendes Betreiben der Impeller-Blutpumpe durch Hinzufügen eines pulsierenden Drehzahlanteils zu einer ersten konstanten Drehzahl, wobei der pulsierende Betrieb der Impeller-Blutpumpe nach Frequenz und Amplitude so eingestellt wird, dass der Verlauf des Transmembrandrucks einem vorgegebenen Verlauf des Transmembrandrucks folgt.

Bei dem Transmembrandruck handelt es sich um den Druckabfall über die Dialysatormembran, die den Dialysator in eine Blutkammer und eine Dialysierflüssigkeitskammer unterteilt. Dieser Druckabfall kann nach diesem Aspekt der Erfindung gezielt durch die entsprechende Betriebsweise der Impeller-Blutpumpe beeinflusst werden. Dabei ist es einerseits möglich, einen möglichst konstanten Druckverlauf und andererseits auch einen stark pulsierenden Druckverlauf einzustellen.

Vor dem Hintergrund der obigen Erläuterungen umfasst die vorliegende Erfindung eine Blutbehandlungsvorrichtung konfiguriert zur Durchführung einer Hämodialyse und/oder Hämofiltration und/oder einer Hämodiafiltration mit einer Steuer- und Recheneinheit, die konfiguriert und/oder programmiert ist, eine Impeller-Blutpumpe anzusteuern. Dabei ist vorgesehen, dass die Impeller-Blutpumpe durch Hinzufügen eines pulsierenden Drehzahlanteils zu einer ersten konstanten Drehzahl pulsierend betrieben wird, wobei die Steuer- und Recheneinheit für einen solchen Betrieb konfiguriert und/oder programmiert ist.

Die Steuer- und Recheneinheit kann dabei so konfiguriert und/oder programmiert sein, dass die Pumpenpulsation ausgehend von gemessenen Druckpulswellen im extrakorporalen Blutkreislauf vorgegeben wird.

Die Blutbehandlungsvorrichtung kann weiterhin einen Drucksensor aufweisen oder mit einem Drucksensor verbindbar sein, über welchen sie den Verlauf einer Druckamplitude an mindestens einer Druckmessstelle des extrakorporalen Blutkreislaufs misst, wobei der gemessene Verlauf der Druckamplitude in einen Soll-Wert für die Steuerung des pulsierenden Drehzahlanteils der Impellerpumpe eingeht, wobei das Messsignal der Druckmessstelle gegebenenfalls durch einen Tiefpass gefiltert und/oder skaliert wird.

Die Steuer- und Recheneinheit ist konfiguriert und/oder programmiert, dass der pulsierende Betrieb der Impeller-Blutpumpe mit dem Verlauf einer durch Herzkontraktion verursachten Druckpulswelle des Patienten im extrakorporalen Blutkreislauf synchronisiert wird. Bevorzugt erfolgt dies durch Heranziehen eines den Patientenpuls kennzeichnenden Parameters.

Weiterhin kann die Steuer- und Recheneinheit so konfiguriert und/oder programmiert sein, dass der pulsierende Betrieb der Impeller-Blutpumpe mit dem Verlauf der durch Herzkontraktion verursachten Druckpulswellen des Patienten im extrakorporalen Blutkreislauf synchronisiert wird, so dass die Fistelrezirkulation des Blutes im extrakorporalen Blutkreislauf ein Minimum annimmt.

Weiterhin kann die Steuer- und Recheneinheit konfiguriert und/oder programmiert sein, folgendes Verfahren durchzuführen:
- Betreiben der extrakorporalen Blutbehandlungsvorrichtung mit dem extrakorporalen Blutkreislauf für eine Hämodialyse und/oder Hämofiltration und/oder Hämodiafiltration, durch Ansteuerung der Impeller-Blutpumpe zum Betrieb mit einer ersten konstanten Drehzahl,
- Bestimmen eines den Patientenpuls kennzeichnenden Parameters
- Pulsierendes Betreiben der Impeller-Blutpumpe durch Hinzufügen eines pulsierenden Drehzahlanteils zu einer ersten konstanten Drehzahl, wobei durch Heranziehen des den Patientenpuls kennzeichnenden Parameters der pulsierende Betrieb der Impeller-Blutpumpe mit dem Verlauf der durch Herzkontraktion verursachten Druckpulswellen des Patienten im extrakorporalen Blutkreislauf synchronisiert wird.

Weiterhin kann die Steuer- und Recheneinheit konfiguriert und/oder programmiert sein, folgendes Verfahren durchzuführen:
- Betreiben der extrakorporalen Blutbehandlungsvorrichtung mit dem extrakorporalen Blutkreislauf für eine Hämodialyse und/oder Hämofiltration und/oder Hämodiafiltration, durch Ansteuerung der Impeller-Blutpumpe zum Betrieb mit einer ersten konstanten Drehzahl,
- Messen mindestens eines ersten Verlaufs der Druckamplitude an mindestens einer Druckmessstelle des extrakorporalen Blutkreislaufs und
- Extrahieren des Verlaufs mindestens einer durch Herzkontraktion verursachten Druckpulswelle des Patienten im extrakorporalen Blutkreislauf aus dem ersten Verlauf der gemessenen Druckamplitude.

Alternativ kann es sich bei dem den Patientenpuls kennzeichnenden Parameter um Messwerte aus EKG, Pulsuhr, Blutdruckmanschette, Ultraschallmessung und/oder Flussmessung handeln. Bevorzugt bestimmt die Steuer- und Recheneinheit dabei mindestens einen dieser Parameter, oder weist eine Schnittstelle auf, über welche sie mindestens einen dieser Parameter erhält.

Weiterhin kann die Steuer- und Recheneinheit konfiguriert und/oder programmiert sein, folgendes Verfahren durchzuführen:
- Messen des Transmembrandrucks in der extrakorporalen Blutbehandlungsvorrichtung und
- pulsierendes Betreiben der Impeller-Blutpumpe durch Hinzufügen eines pulsierenden Drehzahlanteils zu einer ersten konstanten Drehzahl, wobei der pulsierende Betrieb der Impeller-Blutpumpe nach Frequenz und Amplitude so eingestellt wird, dass der Verlauf des Transmembrandrucks einem vorgegebenen Verlauf des Transmembrandrucks folgt.

Die Blutbehandlungsvorrichtung der vorliegenden Erfindung weist eine Antriebseinheit für die Impellerblutpumpe auf, wobei die Steuer- und Recheneinheit konfiguriert und/oder programmiert ist zum Steuern und/oder Regeln der Antriebseinheit für die Impeller-Blutpumpe.

Die Steuer- und Recheneinheit kann weiterhin konfiguriert und/oder programmiert sein zum Auswerten der Messsignale der mindestens einen Druckmessstelle des extrakorporalen Blutkreislaufs.

Bevorzugt erfolgt die Auswertung und/oder Ansteuerung durch die erfindungsgemäße Steuer- und Recheneinheit dabei automatisch.

Weiterhin kann die Steuer- und Recheneinheit dabei konfiguriert und/oder programmiert sein, um eines der oben dargestellten Verfahren durchzuführen, insbesondere automatisch durchzuführen. Weiterhin kann die Steuer- und Recheneinheit dabei so arbeiten, wie dies oben im Hinblick auf das Verfahren dargestellt wurde. Unter einem Verlauf eines den Patientenfluss kennzeichnenden Parameters soll in der vorliegenden Patentanmeldung eine kontinuierliche Erfassung eines Messwerts des den Patientenpuls kennzeichnenden Parameters oder eine phasenweise Erfassung eines solchen Messwerts oder eine sequenzielle Erfassung eines solchen Messwerts, insbesondere auch wiederholte Einzelmessungen und Messungen "von-Peak-zu-Peak" verstanden werden.

Weitere Merkmale, Einzelheiten und Vorteile der Erfindung ergeben sich aus der folgenden Beschreibung einer bevorzugten Ausführungsform zur Erläuterung der im Blutkreislauf auftretenden Druckpulswellen in den beigefügten Figuren. Es zeigen:
- Figur 1:: die gemessenen Druckverläufe des venösen und arteriellen Druckverlaufs an einen mittels einer okkludierenden Rollenpumpe betriebenen extrakorporalen Blutkreislaufs,
- Figur 2:: die gemessenen Druckverläufe des venösen und arteriellen Blutverlaufs an einem mittels einer Impeller-Blutpumpe betriebenen extrakorporalen Blutkreislaufs,
- Figur 3:: den Verlauf des Fistelflusses und des Flusses einer okkludierenden Rollen-Blutpumpe und
- Figur 4:: den Verlauf des Fistelflusses und des Flusses einer Impellerpumpe oder Zentrifugalpumpe.

Eine extrakorporale Blutbehandlungsvorrichtung nach dem Ausführungsbeispiel entspricht dem Aufbau, wie er anhand der DE 10 2009 060 668 A beschrieben ist. Auf eine ausführliche Wiederholung dieser Beschreibung wird an dieser Stelle verzichtet, da es sich hier um einen Standardaufbau handelt.

Wesentlich bei der erfindungsgemäßen Ausführungsvariante der extrakorporalen Blutbehandlungsvorrichtung ist eine Steuer- und Recheneinheit und ein maschinenseitiger Antrieb für eine Impeller-Blutpumpe. Die Impeller-Blutpumpe besteht aus einem Gehäuse mit Impeller und ist vorzugsweise Bestandteil des extrakorporalen Blutschlauchsatzes, der insbesondere als Disposable-Blutkassette ausgeführt ist, wobei der extrakorporale Blutschlauchsatz zur Ankopplung an die extrakorporale Blutbehandlungsvorrichtung konfiguriert ist. Des Weiteren weist die Blutbehandlungsmaschine mindestens einen Drucksensor auf, der zum Ankoppeln an eine Druckmessstelle des extrakorporalen Blutschlauchsatzes konfiguriert ist. Der Drucksensor und die Impeller-Blutpumpe stehen mit der Steuer- und Recheneinheit in Verbindung.

Alternativ kann hier auch - wie im Fall von integrierten RFID-Drucksensoren am Disposable-Blutschlauchsatz - als Verbindung zu der Steuer- und Recheneinheit eine drahtlose Übertragung verwendet werden. Üblicherweise sind an einem extrakorporalen Blutschlauchsatz zumindest eine arterielle Druckmessstelle und eine venöse Druckmessstelle vorhanden. Es kommt für die Ausführung der vorliegenden Erfindung jedoch nicht darauf an, wo sich der mindestens eine Drucksensor am extrakorporalen Blutschlauchsatz befindet, da sich aufgrund eines Herzdruckpulses überall im extrakorporalen Blutkreislauf die Amplitude des gemessenen Drucks ändert und damit an jeder Stelle des extrakorporalen Blutkreislaufs gemessen werden kann.

Die Steuer- und Recheneinheit gemäß der vorliegenden Erfindung weist einen Datenspeicher auf, in dem ein Computerprogramm abgespeichert ist. Der Programmcode des Computerprogramms ist programmiert, um die Impeller-Blutpumpe anzusteuern und die Drucksignale des mindestens einen Drucksensors auszuwerten und abzuspeichern.

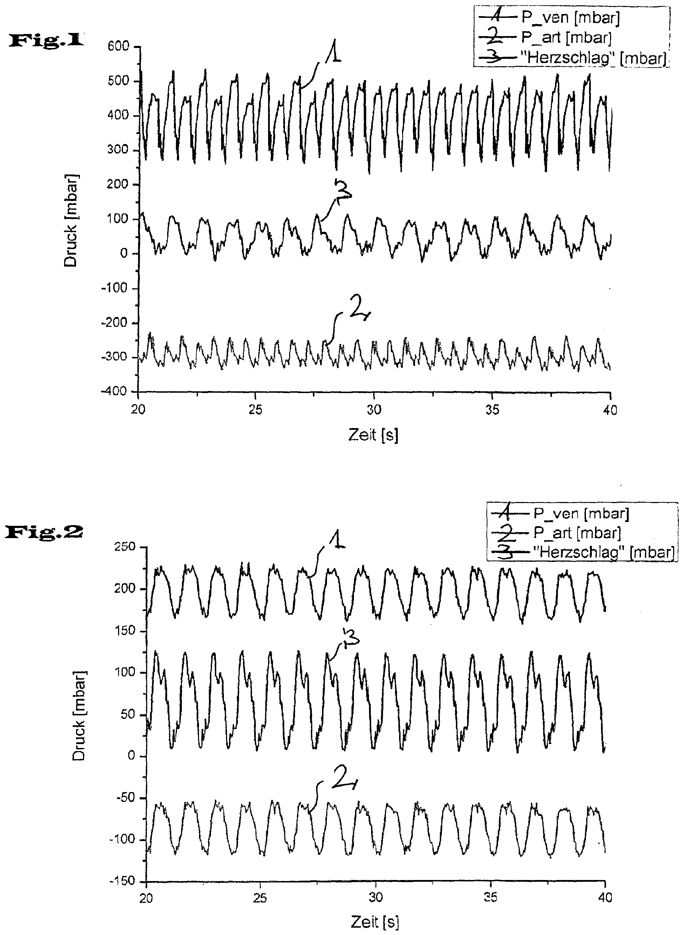

Die Funktionsweise der Erfindung kann anhand des Kurvenverlaufs gemäß der Figuren 1 und 2 näher erläutert werden.

Die Figur 1 zeigt die am extrakorporalen Blutkreislauf gemessenen Druckverläufe des venösen (Kurve 1) und des arteriellen (Kurve 2) Drucks, wobei eine konventionelle peristaltische Blutpumpe zum Betrieb des extrakorporalen Blutkreislaufs verwendet wurde. Die mittlere (mit 3 bezeichnete) Kurve zeigt den korrespondierenden gemessenen Druckverlauf des Druckpulses vom Herz des Patienten, der anderweitig gemessen wurde und der nur zum Vergleich in derselben Grafik dargestellt ist. Es wird hier deutlich, dass die starken Druckpulse der peristaltischen Druckpumpe das Drucksignal dominieren und hinsichtlich der Amplitude und Frequenz stark verfälschen. Anhand der Druckmesskurve des venösen Drucks ist besonders gut zu erkennen, dass die Druckpulse der peristaltischen Blutpumpe, deren Frequenz aufgrund des geförderten Blutflusses im extrakorporalen Blutfluss festliegt, zwangsläufig nicht synchron zu dem Patientenpuls verläuft. Es ist auch eine Schwebung im Drucksignal erkennbar. Insgesamt ist ein solches Drucksignal nicht geeignet, ohne Weiteres als Grundlage zum Extrahieren des Verlaufs der durch Herzkontraktion verursachten Druckpulswellen des Patienten zu dienen.

Das zweite Diagramm gemäß Figur 2 zeigt nun die gemessenen Druckverläufe des venösen (Kurve 1) und des arteriellen (Kurve 2) Drucks für einen extrakorporalen Blutkreislauf, der mit einer Impeller-Blutpumpe betrieben wurde. Die mittlere Kurve (Kurve 3) zeigt wiederum den korrespondierenden Druckverlauf des Druckpulses vom Herz des Patienten, der anderweitig gemessen wurde und nur zum Vergleich in derselben Grafik dargestellt ist. Die gemessenen Pulse der arteriellen und der venösen Druckmessung verlaufen hier nun synchron mit denen der gemessenen Herzpulse. Die gemessenen Herzpulse sind unverfälscht. Somit kann aus dem am extrakorporalen Blutkreislauf gemessenen arteriellen und/oder venösen Druckverlauf zuverlässig der Verlauf der durch Herzkontraktion verursachten Druckpulswellen des Patienten extrahiert werden.

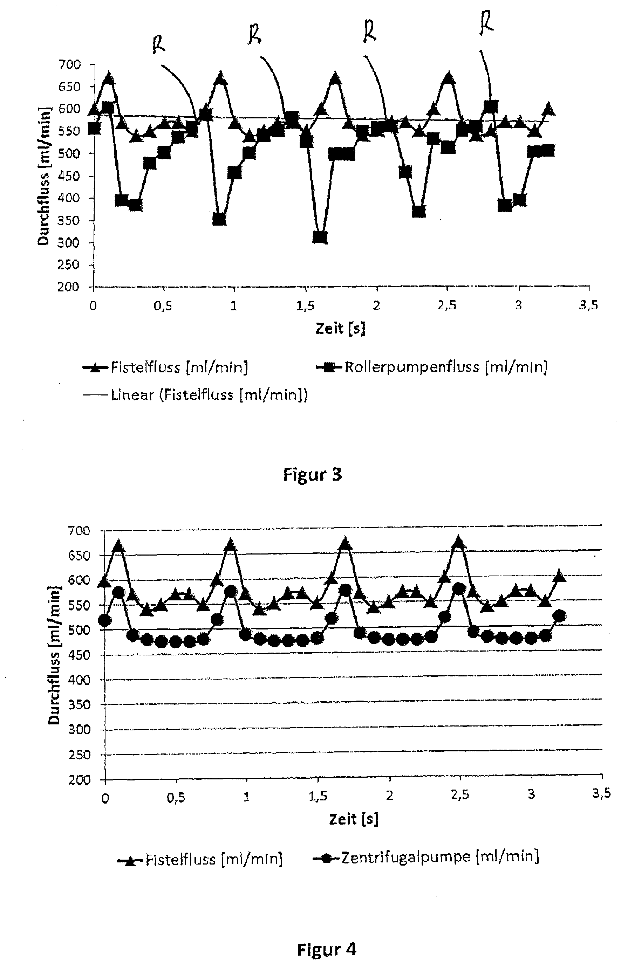

Figur 3 zeigt beispielhaft beim Betrieb eines extrakorporalen Blutkreislaufs die Verläufe des Fistelflusses und des Flusses einer okkludierenden Rollen-Blutpumpe. Der Verlauf des Fistelflusses weist Phasen hoher Fistelzirkulation auf, die mit "R" gekennzeichnet sind. Die Pulsationsfrequenz der Rollen-Blutpumpe ist untrennbar mit dem Durchfluss verknüpft, der wiederum vorgegeben wird. Die Entnahme von Blut aus dem Patientenzugang (Fistel oder Shunt oder Graft) ist daher nicht angepasst an das pulsatile Angebot des Blutflusses in dem Patientenzugang.

Figur 4 zeigt beispielhaft beim Betrieb eines extrakorporalen Blutkreislaufs die Verläufe des Fistelflusses und des Flusses einer Impellerpumpe oder Zentrifugalpumpe. Der Verlauf des Flusses der Impeller- oder Zentrifugalpumpe erfolgt zumindest im Wesentlichen synchron mit dem Verlauf des Fistelflusses. Die Synchronisation der Pulsation der Impeller- oder Zentrifugalpumpe kann dabei die Pulsationsfrequenz und/oder die Amplitude der Pulse umfassen und unabhängig vom vorgegebenen mittleren Blutfluss im extrakorporalen Blutfluss gesteuert und/oder geregelt werden. Die Synchronisation kann anhand des Peaks des Patientenpulses und/oder unmittelbar anhand des gemessenen Drucksignals erfolgen.

Im Rahmen der vorliegenden Erfindung kann insbesondere folgendes Messprinzip eingesetzt werden:
Die Druckpulskurve der Herzpulse wird im extrakorporalen Blutkreislauf (EBK) gemessen. Da das System erfindungsgemäß ohne okkludierende Komponenten, insbesondere ohne peristaltische Pumpen auskommt, ist es als offenes System ausgeprägt, d.h. die Druckpulse des Herzens gelangen durch beide Patientenzugänge in den EBK und überlagern sich dort. Es stellt sich somit ein Summensignal ein, welches nicht oder sehr wenig durch (Druck-)Aktuatoren des EBK selbst gestört ist. Durch das offene Systemkonzept kann der Drucksensor an jeder beliebigen Stelle im System angeordnet sein. Beispielsweise kann ein Drucksensor in der venösen Tropfkammer verwendet werden.

Weiterhin erzeugt die Impellerpumpe oder eine Zentrifugalpumpe anders als eine Peristaltikpumpe keine eigenen Druckpulse. Wegen der fehlenden Störsignale aus dem EBK kann das Summensignal aus den vom Herz des Patienten kommenden Signale daher direkt ausgewertet werden. Erfindungsgemäß ist keine Nachverarbeitung des Signals im Sinne einer Transformation in den Frequenzraum und/oder Filtern von Signalanteilen notwendig, welche auf der Pumpe beruhen. Das System kommt ohne Fouriertransformation aus.

Insbesondere muss damit die Extraktion des Verlaufs mindestens einer durch Herzkontraktion verursachten Druckpulswelle des Patienten im extrakorporalen Blutkreislauf aus dem ersten Verlauf der gemessenen Druckamplitude im Sinne der vorliegenden Erfindung keinen zusätzlichen Schritt der Auswertung umfassen. Vielmehr kann als durch Herzkontraktion verursachte Druckpulswelle des Patienten im extrakorporalen Blutkreislauf der Verlaufs der gemessenen Druckamplitude direkt - gegebenenfalls nach einer Tiefpass-Filterung - herangezogen werden.

Dabei erfolgt keine Auswertung des Signals im Hinblick auf die Erkennung bestimmter Zustände.

Die Steuerung der Impellerpumpe wird direkt mittels eines Regelkreises an das - gegen Rauschen eventuell Tiefpass-gefilterte - Herzdruckpuls-Signal gekoppelt. Somit ist die Frequenz der Pumpenmodulation direkt durch das Messsignal vorgegeben.

Die Amplitude der Blutpumpenmodulation kann mit einem konstanten Faktor, z.B. 1, an die Amplitude des Drucksignals gekoppelt sein.

## Patentansprüche

1. Blutbehandlungsvorrichtung zur Durchführung einer Hämodialyse und/oder Hämofiltration und/oder einer Hämodiafiltration mit einer Steuer- und Recheneinheit, die konfiguriert und/oder programmiert ist, eine Impeller-Blutpumpe anzusteuern, wobei die Blutbehandlungsvorrichtung eine Antriebseinheit für die Impellerblutpumpe aufweist, wobei die Steuer- und Recheneinheit konfiguriert und/oder programmiert ist zum Steuern und/oder Regeln der Antriebseinheit für die Impeller-Blutpumpe, wobei die Impeller-Blutpumpe durch Hinzufügen eines pulsierenden Drehzahlanteils zu einer ersten konstanten Drehzahl pulsierend betrieben wird,
**dadurch gekennzeichnet,**
**dass** die Steuer- und Recheneinheit so konfiguriert und/oder programmiert ist, dass der pulsierende Betrieb der Impeller-Blutpumpe mit einem Verlauf der durch Herzkontraktion verursachten Druckpulswellen des Patienten im extrakorporalen Blutkreislauf synchronisiert wird.

2. Blutbehandlungsvorrichtung nach Anspruch 1, wobei die Steuer- und Recheneinheit so konfiguriert und/oder programmiert ist, dass die Pumpenpulsation ausgehend von gemessenen Druckpulswellen im extrakorporalen Blutkreislauf vorgegeben werden.

3. Blutbehandlungsvorrichtung nach Anspruch 1 oder 2, welche einen Drucksensor aufweist oder mit einem Drucksensor verbindbar ist, über welchen sie den Verlauf einer Druckamplitude an mindestens einer Druckmessstelle des extrakorporalen Blutkreislaufs misst, wobei der gemessene Verlauf der Druckamplitude in ein Soll-Signal für die Steuerung des pulsierenden Drehzahlanteils der Impellerpumpe eingeht, wobei das Messsignal der Druckmessstelle bevorzugt direkt als Soll-Wert herangezogen wird, wofür es gegebenenfalls durch einen Tiefpass gefiltert und/oder skaliert wird.

4. Blutbehandlungsvorrichtung nach Anspruch 1, 2 oder 3, wobei die Steuer- und Recheneinheit so konfiguriert und/oder programmiert ist, dass die Synchronisation des pulsierenden Betriebs der Impeller-Blutpumpe mit dem Verlauf der durch Herzkontraktion verursachten Druckpulswellen des Patienten im extrakorporalen Blutkreislauf durch Heranziehen eines den Patientenpuls kennzeichnenden Parameters.

5. Blutbehandlungsvorrichtung nach einem der Ansprüche 1 bis 4, wobei die Steuer- und Recheneinheit konfiguriert und/oder programmiert ist, folgendes Verfahren durchzuführen:
- Betreiben der extrakorporalen Blutbehandlungsvorrichtung mit dem extrakorporalen Blutkreislauf für eine Hämodialyse und/oder Hämofiltration und/oder Hämodiafiltration, durch Ansteuerung der Impeller-Blutpumpe zum Betrieb mit einer ersten konstanten Drehzahl,
- Messen mindestens eines ersten Verlaufs der Druckamplitude an mindestens einer Druckmessstelle des extrakorporalen Blutkreislaufs und
- Extrahieren des Verlaufs mindestens einer durch Herzkontraktion verursachten Druckpulswelle des Patienten im extrakorporalen Blutkreislauf aus dem ersten Verlauf der gemessenen Druckamplitude.

6. Blutbehandlungsvorrichtung nach Anspruch 4, wobei es sich bei dem den Patientenpuls kennzeichnenden Parameter um Messwerte aus EKG, Pulsuhr, Blutdruckmanschette, Ultraschallmessung und/oder Flussmessung handelt.

7. Blutbehandlungsvorrichtung nach einem der Ansprüche 1 bis 6, **dadurch gekennzeichnet**, die Steuer- und Recheneinheit konfiguriert und/oder programmiert ist zum Auswerten der Messsignale der mindestens einen Druckmessstelle des extrakorporalen Blutkreislaufs.

## Claims

1. A blood treatment apparatus for carrying out a hemodialysis and/or hemofiltration and/or hemodiafiltration having a control and processing unit which is configured and/or programmed to control an impeller blood pump, wherein the blood treatment apparatus comprises a drive unit for the impeller blood pump, wherein the control and/or processing unit is configured and/or programmed to control and/or regulate the drive unit for the impeller blood pump, wherein the impeller blood pump is operated in a pulsed manner by adding a pulsating speed portion to a first constant speed,
**characterized in that**
the control and/or processing unit is configured and/or programmed in such a way that the pulsating operation of the impeller blood pump is synchronized with a course of the pressure pulse waves of the patient, which are caused by cardiac contraction, in the extracorporeal blood circuit.

2. The blood treatment apparatus in accordance with claim 1, wherein the control and processing unit is configured and/or programmed in such a way that the pump pulsation is set based upon pressure pulse waves in the extracorporeal blood circuit.

3. The blood treatment apparatus in accordance with claims 1 or 2, which comprises a pressure sensor or can be connected to a pressure sensor, via which it measures the course of a pressure amplitude at at least one pressure measuring site of the extracorporeal blood circuit, wherein the measured course of the pressure amplitude is considered in a target signal for the control of the pulsating speed portion of the impeller pump, wherein the measuring signal of the pressure measuring site is preferably directly taken as a target value, for what it is possibly filtered and/or scaled by a low-pass filter.

4. Blood treatment apparatus in accordance with claims 1, 2 or 3, wherein the control and processing unit is configured and/or programmed in such a way that the synchronization of the pulsating operation of the impeller blood pump with the course of the pressure pulse waves of the patient caused by cardiac contraction in the extracorporeal blood circle is realized by taking into consideration a parameter characterizing the patient pulse.

5. The blood treatment apparatus in accordance with one of claims 1 to 4, wherein the control and processing unit is configured and/or programmed to perform the following method:
- operating the extracorporeal blood treatment apparatus having the extracorporeal blood circuit for hemodialysis and/or hemofiltration and/or hemodiafiltration by controlling the impeller blood pump for operation at a first constant speed;
- measuring at least one first course of the pressure amplitude at at least one pressure measurement site of the extracorporeal blood circuit; and
- extracting the course of at least one pressure pulse wave of the patient, which is caused by cardiac contraction, in the extracorporeal blood circuit from the first course of the measured pressure amplitude.

6. The blood treatment apparatus in accordance with claim 4, wherein the parameter characterizing the pulse of the patient are measuring values from an electrocardiogram, pulse watch, blood pressure cuff, ultrasonic flow meter and/or flow meter.

7. The blood treatment apparatus in accordance with one of claims 1 to 6, **characterized in that** the control and processing unit is configured and/or programmed for evaluating the measured signals of the at least one pressure measurement site of the extracorporeal blood circuit.

## Revendications

1. Dispositif de traitement sanguin destiné à la réalisation d'une hémodialyse et/ou d'une hémofiltration et/ou d'une hémodiafiltration à l'aide d'une unité de commande et de calcul qui est configurée et/ou programmée pour commander une pompe à sang à roue à aubes, le dispositif de traitement sanguin présentant une unité d'entraînement pour la pompe à sang à roue à aubes, l'unité de commande et de calcul étant configurée et/ou programmée pour la commande et/ou la régulation de l'unité d'entraînement pour la pompe à sang à roue à aubes, la pompe à sang à roue à aubes fonctionnant de manière pulsée par ajout, à une première vitesse de rotation constante, d'une proportion de vitesse de rotation pulsée,
**caractérisé en ce**
**que** l'unité de commande et de calcul est configurée et/ou programmée de manière à ce que le fonctionnement pulsé de la pompe à sang à roue à aubes soit synchronisé avec une allure des ondes de pulsation de pression du patient, causées par la contraction du coeur, dans la circulation sanguine extracorporelle.

2. Dispositif de traitement sanguin selon la revendication 1, l'unité de commande et de calcul étant configurée et/ou programmée de manière à ce que la pulsation de la pompe soit définie en partant d'ondes de pulsation de pression mesurées dans la circulation sanguine extracorporelle.

3. Dispositif de traitement sanguin selon la revendication 1 ou 2, lequel présente un capteur de pression ou est raccordable à un capteur de pression par l'intermédiaire duquel il mesure l'allure d'une amplitude de pression à au moins un point de mesure de pression de la circulation sanguine extracorporelle, l'allure mesurée de l'amplitude de pression se transformant en un signal théorique pour la commande de la proportion de vitesse de rotation pulsée de la pompe à roue à aubes, le signal de mesure du point de mesure de pression étant pris en considération de préférence directement en tant que valeur théorique, et étant pour cela éventuellement filtré et/ou ajusté par un passe-bas.

4. Dispositif de traitement sanguin selon la revendication 1, 2 ou 3, l'unité de commande et de calcul étant configurée et/ou programmée de manière à ce que la synchronisation du fonctionnement pulsé de la pompe à sang à roue à aubes avec l'allure des ondes de pulsation de pression du patient, causées par la contraction du coeur, dans la circulation sanguine extracorporelle soit réalisée par prise en compte d'un paramètre caractérisant le pouls du patient.

5. Dispositif de traitement sanguin selon l'une quelconque des revendications 1 à 4, l'unité de commande et de calcul étant configurée et/ou programmée pour réaliser le procédé suivant :
- fonctionnement du dispositif de traitement sanguin extracorporel avec la circulation sanguine extracorporelle pour une hémodialyse et/ou une hémofiltration et/ou une hémodiafiltration par commande de la pompe à sang à roue à aubes pour le fonctionnement avec une première vitesse de rotation constante,
- mesure d'au moins une première allure de l'amplitude de pression à au moins un point de mesure de pression de la circulation sanguine extracorporelle, et
- extraction, à partir de la première allure de l'amplitude de pression mesurée, de l'allure d'au moins une onde de pulsation de pression du patient, causée par la contraction du coeur, dans la circulation sanguine extracorporelle.

6. Dispositif de traitement sanguin selon la revendication 4, le paramètre caractérisant le pouls du patient étant des valeurs de mesure provenant d'un ECG, d'un pulsomètre, d'un brassard de tensiomètre, d'une mesure d'échographie et/ou d'une mesure de flux.

7. Dispositif de traitement sanguin selon l'une quelconque des revendications 1 à 6, **caractérisé en ce que** l'unité de commande et de calcul est configurée et/ou programmée pour évaluer les signaux de mesure de l'au moins un point de mesure de pression de la circulation sanguine extracorporelle.
